Europäisches Patentamt

(19) European Patent Office

Office européen des brevets

(11) Publication number: **0 241 929 B1**

## (12) EUROPEAN PATENT SPECIFICATION

(45) Date of publication of patent specification:
23.10.91 Bulletin 91/43

(51) Int. Cl.⁵: **A01M 1/20,** A01M 13/00,
A61L 9/03

(21) Application number: 87105613.1

(22) Date of filing: 15.04.87

(54) Catalytic heating device for evaporating volatile substances contained in insect repellent or deodorant tablets.

(30) Priority: 16.04.86 IT 2156586 U

(43) Date of publication of application:
21.10.87 Bulletin 87/43

(45) Publication of the grant of the patent:
23.10.91 Bulletin 91/43

(84) Designated Contracting States:
AT BE CH DE FR GB IT LI NL SE

(56) References cited:
EP-A- 0 120 968
WO-A-86/01980
DE-A- 2 946 631
FR-A- 2 215 864
FR-A- 2 482 420

(73) Proprietor: ZOBELE INDUSTRIE CHIMICHE
S.p.A.
Via Fersina, 4
I-38100 Trento (IT)

(72) Inventor: Zobele, Franco
Via Adamello 28
I-38100 Trento (IT)

(74) Representative: Dr. Ing. A. Racheli & C.
Viale San Michele del Carso, 4
I-20144 Milano (IT)

## Description

The present invention concerns catalytic heating devices that are used, for example to volatilize a perfumed substance or a substance such as to drive away pests such as insects etc. Various types of catalytic heating devices are known, but they have some drawbacks. In particular, they always allow a certain amount of unpleasant smelling combustible fumes to escape. Furthermore, many of them are relatively difficult to light, making it necessary to use expensive fuels, as well as to replace the catalytic element frequently.

The aim of the present invention is to create a heating device which does not allow combustible fumes to escape into the environment when it is used.

Other aims are to achieve a simple, functional design, to permit easy lighting of the catalytic element and to guarantee excellent combustion and long life of said element.

The principal and secondary aims have been achieved as specified in the attached claims.

The present invention will now be better described on the basis of an exemplary embodiment which is shown in the attached figures, in which:

Fig. 1 is a partially sectioned, exploded view of the proposed heating device; and

Fig. 2 is a sectional view along plane 2-2 of figure 1 illustrating the heating device in its operating state.

With reference to the drawings it will be noted that the proposed heating device consists of a base 10 containing at a distance a tank 11 in which is inserted a spongy medium 19 which will be soaked with combustible. A channel 17 is thus formed between the base 10 and the tank. In some areas 13 there is a small space between the base 10 and the surface on which it rests so that air can penetrate from these areas 13 and flow upwards in channel 17. This air will be able to pass into the upper parts through large holes 12 in a horizontal wall 14 which connects the base to the tank 11.

A catalytic heating device of the same kind according to the pre-characterizing portion of claim 1 is known from EP-A-120968: There is another disclosed heating device in WO-A-8 601 980 which comprises a metal fuel tank provided in the upper part with a slit shaped opening surrounded by a holding member which holds the burner at a distance above the slit shaped opening. In this kind of structure the combustible vapours can escape from the tank through the free space existing all around the burner which has a cylindrical oblong shape without passing through the burner. Furthermore in the second cited example of the prior art the lighting of the burner is difficult because first the tank is almost completely filled with absorbent material, and there is not space enough for vapour feeding combustion, and second said slit shaped opening, i.e. the useful surface for evaporation, is too small.

The tank 11 ends in a wide mouth 16 which is surrounded by rim 15 which is such as to engage in an airtight manner with cover 20 in which there is a central opening 21. A catalytic element 22 is introduced into this opening 21, said catalytic element being contained in a protective net 23 with a projecting part 23a designed to penetrate into the opening 21 and a flange 23b designed to adhere to the wall of the cover surrounding opening 21. Catalytic element 22 and net 23 are fixed by means of a perforated disk 24 which is inserted into the cover 20 in such a way as to form a seal with it and leave clear opening 24a which matches opening 21. The edges 25 of cover 20 are such as to adhere to the upper rim 15 of the tank forming a seal.

In this way the tank is perfectly airtight except for opening 21 which, however, is closed by catalytic element 22.

Over the cover 20 will be placed a dome 30 comprising a perforated top guard 31, set directly above the catalytic element, insertion windows 32, as well as projecting parts 33 which create a support for the tablets P to be evaporated (shown by alternating dashes and dots). The side walls 34 of the dome are sealed and form a single body with the base 10 when said dome is mounted. The dome 30 is also provided with walls 36 which create a continuous sealed surface with the walls 34, leaving clear only a gap 35 between the bottom of said wall 36 and the upper surface of the tank cover 20. This makes it possible to create a chimney which runs from the open areas 13, into the channel 17, through the holes 12, inside the dome to the gap 35, then licks the catalytic element 22 and finally makes for the tablet set on projecting parts 33 and goes out through the top guard 31. The whole of this path is airtight and extends up the entire height of the heating device thus exploiting a chimney effect. It should be noted that the air which supports combustion can only follow the above path, all other ways being blocked.

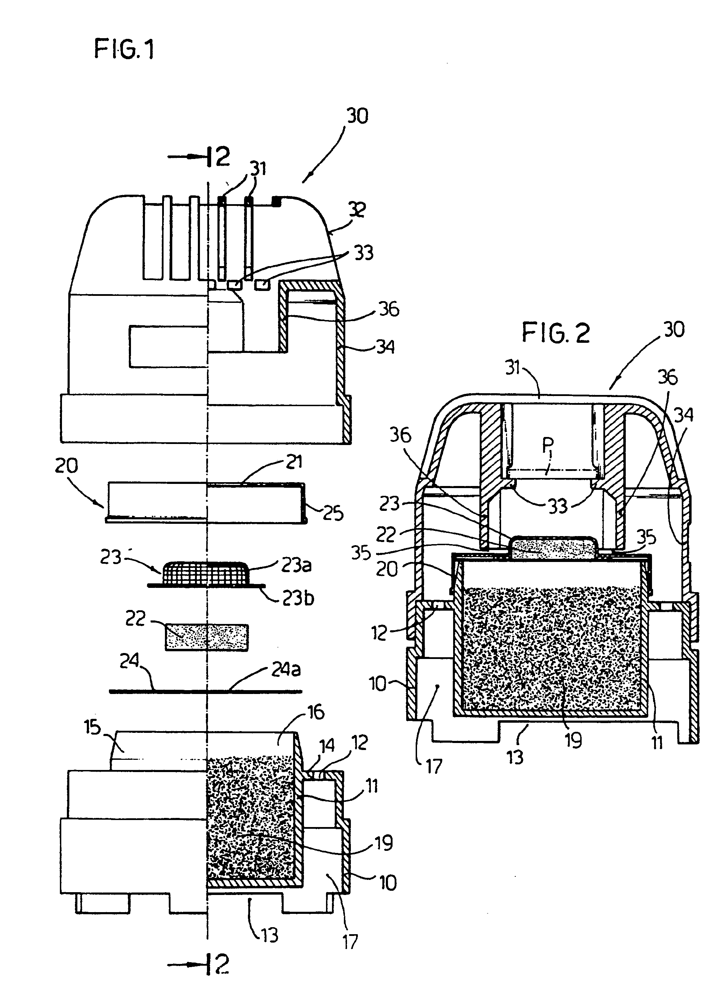

As will be noted from figure 2, after having filled tank 11 with combustible and fitted the top 20 onto it, the combustible fumes will only be able to pass through opening 21. The tank 11 and cover 20 will then be tilted and the catalytic element 22 will be heated, with the flame of a cigarette lighter for example. As soon as element 22 is hot, combustion will continue, being favoured by said catalytic element without any need for a flame. The combustible fumes will continue to burn completely due to the presence of the catalytic element and supported by the chimney effect described above.

The tank can be made of plastic material because it will not be heated. Heating will in fact be limited to catalytic element 22, top 20 and perforated disk 24. The heat will not spread any further since the cover is large enough to cool down before its peripheral surface come into contact with the upper rim 15 of the tank 11. The cover 20 is also efficiently cooled by the circulating combustion air flowing through gap 35.

## Claims

1. A catalytic heating device for evaporating volatile substances contained in a tablet (P), comprising a base (10) and a dome (30) which is, at least during operation, hermetically coupled to the base (10) in such a way that the ensemble base-dome (10,30) forms a single body, a fuel tank (11) and a catalytic element (22) placed in the upper part of the tank (11) through which the combustible vapours pass when the device is working, said catalytic element being reached by external air,
characterised in that the fuel tank (11) is completely housed in the base (10) and in that the catalytic element (22) is reached by the air flow starting from the bottom of the base (10) and flowing upwardly through the interior of said base.

2. A heating device according to claim 1, characterized in that open areas (13) are provided right at the bottom of the base (10), and holes (12) are provided in a linking wall between the base and the tank, only an internal gap (35) being provided between the dome (30) and the base (10) for the passage of said combustion supporting air, so that an air chimney is created in the heating device between the open areas (13), the catalytic element (22), the tablet (P) housed in the dome (30) and a top guard (31), thus favouring combustion.

3. A heating device according to claim 2, characterized in that the gap (35) is envisaged between the inner walls (36) of the dome and a metal cover (20) which closes the mouth (16) of the tank and holds the catalytic element (22).

4. A heating device according to any one of the preceding claims, characterized in that the catalytic element (22) is platinum-coated quartz fibre.

5. A heating device according to any one of the preceding claims, characterized in that the catalytic element (22) is held by a net (23) which is enclosed between a perforated cover (20) and a disk (24) which forms a seal with it.

6. A heating device according to any one of the preceding claims, characterized in that the catalytic element (22) protrudes through an opening (21) in the cover (20).

7. A heating device according to any one of the preceding claims, characterized in that all its parts are of plastic, except for the cover (20), the net (23) and the disk (24).

8. A heating device according to any one of the preceding claims, characterized in that said tank (11) forms a single body with the base (10).

9. A heating device according to any one of the preceding claims, characterized in that the chimney which forms the path for the combustion supporting air is of the same height as the whole heating device.

10. A heating device according to any one of the preceding claims, characterized in that it has a circular simmetry.

## Patentansprüche

1. Katalytisches Heizelement zum Verdampfen von flüchtigen Stoffen, die in einer Platte (P) enthalten sind, mit einer Basis (10) und einer Kuppel (30), die mindestens während des Betriebs luftdicht mit der Basis (10) gekuppelt ist, sodaß die Gruppe Basis-Kuppel (10,30) einen einzigen Körper bildet, mit einem Behälter (11) und einem katalytischen Element (22), das im oberen Teil des Behälters (11) angeordnet ist, durch den die Brennstoffdämpfe strömen, wenn die Vorrichtung in Betrieb ist, wobei Außenluft zum katalytischen Element gelangt,
dadurch gekennzeichnet, daß der Behälter (11) vollständig in der Basis (10) eingeschlossen ist und daß das katalytische Element (22) vom Luftstrom erreicht wird, der vom Boden der Basis (10) ausgeht und das Innere der Basis nach oben durchströmt.

2. Heizelement nach Anspruch 1, dadurch gekennzeichnet, daß offene Bereiche (13) im untersten Teil der Basis (10) und Löcher (12) an einer Verbindungswand zwischen Basis und Behälter vorgesehen sind, daß nur ein Zwischenraum (35) zwischen der Kuppel (30) und der Basis (10) für die Durchleitung der Verbrennungsgase vorgesehen ist, sodaß sich im Heizelement ein Luftkamin zwischen den offenen Bereichen (13), dem katalytischen Element (22), der in der Kuppel (30) angeordneten Platte (P) und einem oberen Schutz (31) bildet, der die Verbrennung begünstigt.

3. Heizelement nach Anspruch 2, dadurch gekennzeichnet, daß der Zwischenraum (35) zwischen Innenwände (36) der Kuppel (30) und einem Metalldeckel (20), der die Öffnung (16) des Behälters schließt und das katalytische Element festhält, vorgesehen ist.

4. Heizelement nach einem der vorstehenden Ansprüche, dadurch gekennzeichnet, daß das katalytische Element (22) aus Quarzfaser mit Platinumhüllung ist.

5. Heizelement nach einem der vorstehenden Ansprüche, dadurch gekennzeichnet, daß das katalytische Element (22) von einem Netz (23) gehalten ist, das sich zwischen einem gelochten Deckel (20) und einer abdichtenden Scheibe (24) erstreckt.

6. Heizelement nach einem der vorstehenden Ansprüche, dadurch gekennzeichnet, daß das katalytische Element (22) aus einer im Deckel (20) angeordneten Öffnung (21) ragt.

7. Heizelement nach einem der vorstehenden Ansprüche, dadurch gekennzeichnet, daß seine sämtlichen Teile mit Ausnahme von Deckel (20), Netz (23) und Scheibe (24) aus Kunststoff sind.

8. Heizelement nach einem der vorstehenden Ansprüche, dadurch gekennzeichnet, daß der Behälter (11) mit der Basis (10) einen einzigen Körper bildet.

9. Heizelement nach einem der vorstehenden Ansprüche, dadurch gekennzeichnet, daß der Kamin für die Verbrennungsgase die gleiche Höhe wie die Höhe des gesamten Heizelementes hat.

10. Heizelement nach einem der vorstehenden Ansprüche, dadurch gekennzeichnet, daß es eine kreisförmige Symmetrie aufweist.

**Revendications**

1. Elément de chauffage catalytique pour évaporer des substances volatiles contenues dans des comprimés (P), comprenant une base (10) et une coupole (30) s'enclenchant hermétiquement, du moins au cours de fonctionnement, sur la base (10) de sorte que l'ensemble base-coupole (10,30) forme un corps unique, un réservoir (11) et un élément catalytique (22) placé dans la partie supérieure du réservoir (11) à travers lequel passent les vapeurs de combustible quand le dispositif fonctionne, de l'air extérieur arrivant audit élément catalytique,
caractérisé en ce que le réservoir (11) est entièrement contenu dans la base (10) et que l'élément catalytique (22) est atteint par le flux d'air qui part du fond de la base (10) et se dirige vers le haut à travers l'intérieur de ladite base.

2. Elément de chauffage selon la revendication 1, caractérisé en ce que sont prévues des zones ouvertes (13) dans la partie extrême inférieure de la base (10) et des trous (12) dans une paroi de liaison entre la base et le réservoir, seul un interstice (35) étant prévu entre la coupole (30) et la base (10) pour le passage de la dite air comburant, de sorte qu'un passage d'air se crée dans l'élément de chauffage entre les zones ouvertes (13), l'élément catalytique (22), le comprimé (P) logé dans la coupole (30) et une protection supérieure (31), favorisant la combustion.

3. Elément de chauffage selon la revendication 2, caractérisé en ce que l'interstice (35) est prévu entre les parois internes (36) de la coupole (30) et un couvercle (20) métallique fermant la boucle (16) du réservoir et retient l'élément catalytique (22).

4. Elément de chauffage selon n'importe laquelle des revendications précédentes, caractérisé en ce que l'élément catalytique (22) est en fibre de quartz recouverte de platine.

5. Elément de chauffage selon n'importe laquelle des revendications précédentes, caractérisé en ce que l'élément catalytique (22) est retenu par un filet (23) qui est compris entre un couvercle (20) troué et un disque (24) d'un seul tenant avec ce dernier.

6. Elément de chauffage selon n'importe laquelle des revendications précédentes, caractérisé en ce que l'élément catalytique (22) fait saillie en dehors d'une ouverture (21) faite dans le couvercle (20).

7. Elément de chauffage selon n'importe laquelle des revendications précédentes, caractérisé en ce que toutes ses parties sont en plastique, sauf le couvercle (20), le filet (23) et le disque (24).

8. Elément de chauffage selon n'importe laquelle des revendications précédentes, caractérisé en ce que ledit réservoir (11) est d'un seul tenant avec la base (10).

9. Elément de chauffage selon n'importe laquelle des revendications précédentes, caractérisé en ce que le passage le long duquel s'étend le parcours de l'air comburant a une hauteur égale à la hauteur de tout l'élément de chauffage.

10. Elément de chauffage selon n'importe laquelle des revendications précédentes, caractérisé en ce qu'il présente une symétrie circulaire.

# FIG.1

# FIG. 2